# EUROPEAN PATENT APPLICATION

(11) **EP 1 310 266 A1**
(43) Date of publication of application: **14.05.2003**
(21) Application number: 02257801.7
(22) Date of filing: 13.11.2002
(51) Int. Cl.: A61L 15/32, A61L 15/44, A61L 27/22, A61L 27/54, A61L 29/04, A61L 29/16, A61L 31/04, A61L 31/16

(54) **Medical devices that stimulate growth factor production**

(30) Priority: 13.11.2001 US 8430
(71) Applicant: ST. JUDE MEDICAL, INC., St. Paul, MN 55117 (US)
(72) Inventor: Ogle, Matthew F., Oronoco, Minesota 55960 (US); McConico, Andrea L., Fridley, Minnesota 55432 (US)
(74) Representative: Wakerley, Helen Rachael

(57) **Abstract**

Medical devices are described that have a releasable quantity of a stimulation compound that stimulates production of VEGF. The stimulation compound can be a polypeptide, such as hypoxia-inducible factor 1. Suitable stimulation compounds stimulate transcription of VEGF. Medical devices of particular interest include, for example, heart valve prostheses, vascular prostheses and vascular stents.

## Description

### FIELD OF THE INVENTION

The invention relates to medical devices that include compounds to stimulate production of growth factors. Furthermore, the invention relates to heart valve prostheses incorporating a biocompatible material that includes a stimulation compound to stimulate production of growth factors.

### BACKGROUND OF THE INVENTION

Various medical articles have been designed particularly for contact with a patient's body fluids. This contact can be sufficiently long such that surface interactions between the medical article and the patient's blood and/or tissue become significant. For example, the host interaction with the medical article may lead to degradation, such as calcification of the medical article. Relevant medical articles include, for example, catheters and prostheses.

Prostheses, i.e., prosthetic devices, are used to repair or replace damaged or diseased organs, tissues and other structures in humans and animals. Prostheses must be generally biocompatible since they are typically implanted for extended periods of time. Prostheses can be constructed from natural materials, synthetic materials or a combination thereof. For example, prostheses, such as mechanical heart valve prostheses, are manufactured from biocompatible materials, such as pyrolytic carbon coated graphite and polyester. Bioprosthetic heart valves typically are derived from pig aortic valves or are manufactured from other biological materials, such as bovine pericardium. Catheters include percutaneous devices that penetrate the skin to provide access to a body system.

Mechanical heart valves generally include an occluder that pivots within an orifice ring, through which blood flows. Pivoting of the occluder opens and closes the valve. Although mechanical heart valves have the advantage of proven durability through decades of use, they are associated with a potential for blood clotting on or around the prosthetic valve. Blood clotting can lead to acute or subacute closure of the valve. For this reason, patients with implanted mechanical heart valves remain on anticoagulants after implantation of the valve. Anticoagulants impart a potential risk of bleeding complications and cannot be taken safely by certain individuals. Occluders and orifice rings of mechanical heart valves can be formed from materials, such as pyrolytic carbon, that reduce the risk of blood clotting by the nature of their relative thromboresistance.

In addition to heart valve prostheses formed with rigid occluders, heart valve prostheses can be constructed from tissue or flexible polymer materials. The tissue or polymer leaflets generally flex to open and close the valve. Thrombosis can be a concern associated with polymer heart valves. Tissue-derived prosthetic heart valves generally have blood flow characteristics and surface properties that provide a high degree of thromboresistance without the need for anticoagulant therapy. Therefore, thrombosis or thromboembolism and bleeding complications are less likely to occur with a tissue valve than with mechanical heart valves. Unfortunately, prosthetic tissue heart valves are limited by a tendency to fail. Replacement of a degenerating valve prosthesis subjects the patient to additional surgical risk. Valve degeneration is particularly rapid in young patients and during pregnancy.

Calcification, i.e., the deposition of calcium salts, especially calcium phosphate (hydroxyapatite), appears to be a major cause of the degeneration of tissue-based valves. Flexible polymer heart valves also tend to fail due to calcification and subsequent degeneration. Calcification can affect the performance and structural integrity of medical articles constructed from susceptible materials, especially over extended periods of time. Other prostheses made from natural and/or synthetic materials may also display clinically significant calcification.

Native heart valve tissue with viable cells has natural protection against calcification and provides desirable thromboresistant properties. Endothelial cells that coat the blood contacting surfaces of a native valve provide a barrier against calcification. These cells also protect against infection and provide active thromborcsistance.

### SUMMARY OF THE INVENTION

In a first aspect, the invention pertains to a medical device comprising a stimulation compound that stimulates production of VEGF. The medical device can be, for example, an implantable medical device, a catheter, a dressing or a surgical instrument. In some embodiments, the stimulation compound is releasable.

In another aspect, the invention pertains to a method for producing a medical device, the method including the association of a stimulation compound with a biocompatible material. Approaches for associating the stimulation compound include, for example, direct association, chemical bonding, adhesive bonding, and incorporating the stimulation compound into the matrix of the biocompatible material.

### BRIEF DESCRIPTION OF THE DRAWINGS

Fig. 1 is a perspective view of a bileaflet mechanical heart valve prosthesis.

Fig. 2 is a tissue-based aortic heart valve prosthesis.

Fig. 3 is a perspective view of a vascular graft prosthesis.

Fig. 4 is a side view of the vascular graft prosthesis of Fig. 3 attached to a blood vessels at each end of the prosthesis.

Fig. 5 is a top view of an annuloplasty ring.

Fig. 6 is a perspective view of a support stent for a stented heart valve prosthesis with flexible leaflets.

Fig. 7 is a side view of a vascular stent.

### DETAILED DESCRIPTION OF THE INVENTION

Medical devices, as described herein, incorporate improved biocompatible materials that include associated compounds that stimulate vascular cndothelial growth factor (VEGF) production. Thus, the presence of the stimulation compounds promotes the proliferation of cells in the vicinity of the medical device. The proliferation of endothelial cells near the medical device and/or the colonization of the medical device by endothelial cells can improve the flow in the vicinity of the medical device and may decrease calcification of the medical device. The stimulation compound can be releasably associated with the material such that the stimulation compound is gradually released into the fluid near the medical device. The stimulation compounds can be associated with natural and/or synthetic biocompatible materials.

Relevant medical articles include devices that contact a person's body fluids and tissues for varying lengths of time, for example, prostheses, catheters and surgical instruments. Prostheses, i.e., prosthetic articles, are used to repair or replace damaged or diseased organs, tissues and other structures in a patient. Prostheses generally must be biocompatible since they are typically implanted for extended periods of time. Generally, the patient is an animal, preferably a mammal, such as a human. Medical devices of particular interest include, for example, heart valve prostheses and other vascular and cardiovascular prostheses. Suitable heart valve prostheses include heart valve prostheses with rigid occluders and prostheses with flexible leaflets. The medical devices include a biocompatible material that includes an associated bioactive molecule that can stimulate VEGF production. These bioactive molecules capable of stimulating VEGF production are referred to herein as stimulation compounds.

The biocompatible materials generally are non-toxic, non-carcinogenic and do not induce hemolysis or a significant immunological response. The biocompatible material with associated stimulation compounds can include tissue material and/or synthetic material. Suitable synthetic materials include, for example, polymers, ceramics, carbon solids, metals and the like. Suitable tissue materials include, for example, tissues and tissue derived matrices. The stimulation compound can be associated with one or more materials in the medical device at one or more locations within the medical device. In some embodiments, the stimulation compound is associated with the entire medical device.

Hypoxia inducible factor-1 (HIF-1) is a heterodimer with two protein subunits, hypoxia inducible factor 1-alpha (HIF-1α) and hypoxia inducible factor 1-beta (HIF-1β). HIF-1β is also known as aryl hydrocarbon receptor nuclear translocator and can dimerize with other protein subunits. HIF-1α is able to stimulate production of VEGF. HIF-1α is a protein factor that is found to be expressed in response to hypoxia. Hypoxia is a lack of an adequate amount of oxygen. Hypoxia can elicit responses at systemic, local and cellular levels to increase oxygen delivery or activate alternative metabolic pathways that do not require oxygen. Hypoxia-inducible proteins include a variety of proteins in addition to VEGF. Transcription of the genes encoding these proteins is activated by HIF-1. Specifically, HIF-1 activates transcription of genes for VEGF, erythropoictin, heme oxygenase-1, inducible nitric oxide synthase, and glycolytic enzymes. Hypoxia response is described further in U.S. Patent 6,124,131 to Semenza, entitled "Mutant Hypoxia Inducible Factor- 1 HIF-1," incorporated herein by reference.

Medical devices arc formed such that a biocompatible material within the device includes HIF-1 and/or HIF-1α. "HIF" is used herein to refer collectively to HIF-1, HIF-1α, derivatives thereof that are functionally active and combinations thereof. In some embodiments, the HIF is gradually released into the neighboring environment. Various approaches can be used for the controlled release of the stimulation compound, as described further below. Release of the HIF stimulates proliferation of endothelial cells in the vicinity of the medical device through a chain reaction of responses. As a result, endothelialization of the materials in the medical device may take place. The HIF can be placed in contact with the substrate that is desired for endothelialization, and/or the HIF can be associated with a material near the material to be endothelialized. The attraction of viable cells to a substrate of the prosthesis should improve the long-term viability of the medical device by decreasing or eliminating available surface areas that are prone to calcification. In addition, the presence of endothelial cells can reduce the incidence of other undesirable side effects, such as restenosis and thrombosis.

The stimulation compounds can be associated with the biocompatible material before, during or after the biocompatible material is formed into the prosthesis or other medical device, if assembly is required. A prosthesis with associated stimulation compounds can be implanted into the patient such that the patient's cells are stimulated into producing VEGF. VEGF production correspondingly stimulates proliferation of endothelial cells in the vicinity of the medical device. These endothelial cells can become associated with one or more of the materials in the medical device. In addition, other elements of the vascularization response stimulated by HIF may be beneficial with respect to colonization of the medical device by viable cells from the patient and/or other desirable modifications of the environment in the vicinity of the medical device.

The HIF and/or other stimulation compounds direct natural processes that encourage cellular activity and vascularization near the medical device without the effort associated with in vitro manipulation of cells. Nevertheless, in alternative embodiments, the biocompatible material or the entire medical device can be contacted with a solution in vitro containing endothelial cells to populate the substrate with endothelial cells. In some embodiments, the endothelial cells for in vitro processes are obtained from the patient who will receive the medical device. To obtain the cells, the endothelial cells generally can be harvested from the patient's blood or bone marrow.

The presence of the stimulation compound creates a response effect that stimulates proliferation of endothelial cells in the vicinity of the medical device. These endothelial cells may or may not colonize the medical device. Whether or not the endothelial cells colonize the medical device, the proliferation of endothelial cells in the vicinity of the medical device can be beneficial with respect to improving the flow properties of the medical device. Furthermore, the presence of endothelial cells in the vicinity of the medical device can be effective to reduce or eliminate the colonization of the biocompatible material by microorganisms. Incorporation of a stimulation molecule capable of stimulating VEGF production near the surface of a medical device, such as a heart valve prosthesis, or a portion thereof, could reduce the risk of thrombosis and the long-term need for anticoagulation therapy. Also, since endothelial cells can stimulate the clean up of non-viable cells and prevent nucleation of calcific deposits, modifications that promote colonization of the tissue by these cells can reduce calcification and improve prosthesis durability. In addition, the presence of endothelial cells may inhibit undesirable responses, such as processes that result in restenosis.

### Medical Devices

Relevant medical devices generally include a biocompatible material suitable for contacting a patient's body fluids and tissues. The biocompatible material may be suitable as a location for cellular attachment. Generally, these medical devices are prostheses or components thereof designed for implantation into or onto a patient for extended periods of time, although percutaneous devices, surgical instruments and dressing may be appropriate medical devices. Prostheses include, for example, artificial hearts, heart valves, other artificial organs, annuloplasty rings, pericardial patches, vascular, cardiovascular and structural stents, ocular devices, vascular grafts or conduits, pledgets, suture, permanently in-dwelling percutaneous devices, vascular or cardiovascular shunts, dermal grafts for wound healing, and surgical patches. Biomedical devices that are designed to dwell for extended periods of time within a patient are also suitable to include stimulation compounds. These devices include, for example, Hickman catheters.

Percutaneous devices include, without limitation, catheters of various types, cannulas, bronchial tubes, intratracheal tubes, Foley catheters, and drainage tubes such as chest tubes. Catheters can be used for accessing various body systems such as the vascular system, the gastrointestinal tract, or the urinary system. Surgical instruments include devices such as forceps, retractors, needles, gloves, and catheter cuffs. Dressings include, without limitation, skin grafts, burn dressings, and wound dressings of all types.

While the biocompatible material with associated stimulation compounds can be used in any of the medical devices described above, a few medical devices are of particular interest. Such devices of particular interest include, for example, prostheses that contact systems in the body that are lined with endothelial cells, such as vascular prostheses and cardiovascular prostheses. More specifically, medical devices of interest include, for example, heart valve prostheses, components of heart valve prostheses, vascular stents, urinary stents, annuloplasty rings, pacemaker components and implantable electrical leads.

A heart valve prosthesis can be designed as a replacement for any heart valve, e.g., an aortic valve, a mitral valve, a tricuspid valve, or a pulmonary valve. Some heart valve prostheses have rigid occluders that pivot to open and close the valve while other heart valve prostheses have flexible leaflets of polymer or tissue that open and close in response to pressure differentials. Heart valves with flexible polymer leaflets include a support structure that supports the flexible leaflets. If the support structure is sufficiently rigid to maintain leaflet function against the pressures in the blood flow, the support structure is called a stent. If the support structure is flexible, the valve is called a stentless valve, and the support structure is secured to other natural or artificial structures to maintain valve function. With any valved prosthesis, the valve generally acts as a one-way check valve that opens for fluid flow in one direction and closes to limit fluid flow in the opposite direction.

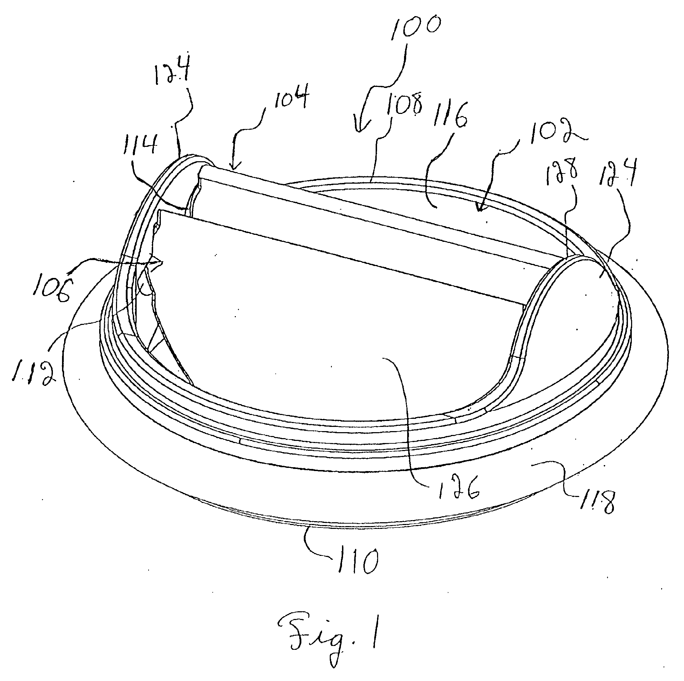

An embodiment of a bileaflet mechanical heart valve prosthesis 100 is shown in Fig. 1. Heart valve prosthesis 100 includes an orifice ring 102 which retains two rigid occluders 104, 106. Orifice ring 102 has an upstream surface 108 and a downstream surface 110. Occluders 104, 106 rotate at pivots 112, 114 and two additional opposed pivots symmetrically positioned on the inner luminal surface 116 of orifice ring 102 (not shown). Inner luminal surface 116 of orifice ring 102 forms a flow path through the valve that can be opened or closed through the pivoting of occluders 104, 106. A sewing cuff 118 is placed around orifice ring 102 to facilitate attachment to the patient's tissue during implantation of the valve. In the embodiment shown in Fig. 1, an orifice ring 102 has a pivot guard structure 124, which projects upstream of the plane of orifice ring 102.

Blood flows through the prosthesis in an effectively unidirectional way. Occluders 104, 106, shown in the open position in Fig. 1, pivot in response to forces imparted by the blood during the cardiac cycle. Occluders 104, 106 cyclically close to effectively block back flow through the valve lumen. In the closed position, downstream surfaces of occluders 104, 106 block back flow through the valve. Downstream surface 126 of occluder 106 is shown in Fig. 1. Occluders 104, 106 similarly assume an open position to allow forward flow through the valve lumen. All or a portion of prosthesis 100 can be formed from a biocompatible material with an associated stimulation compound. Features of the invention are suitable for use with other mechanical valve designs including, for example, single leaflet valves, valves with more than two leaflets and valves with different pivoting structures.

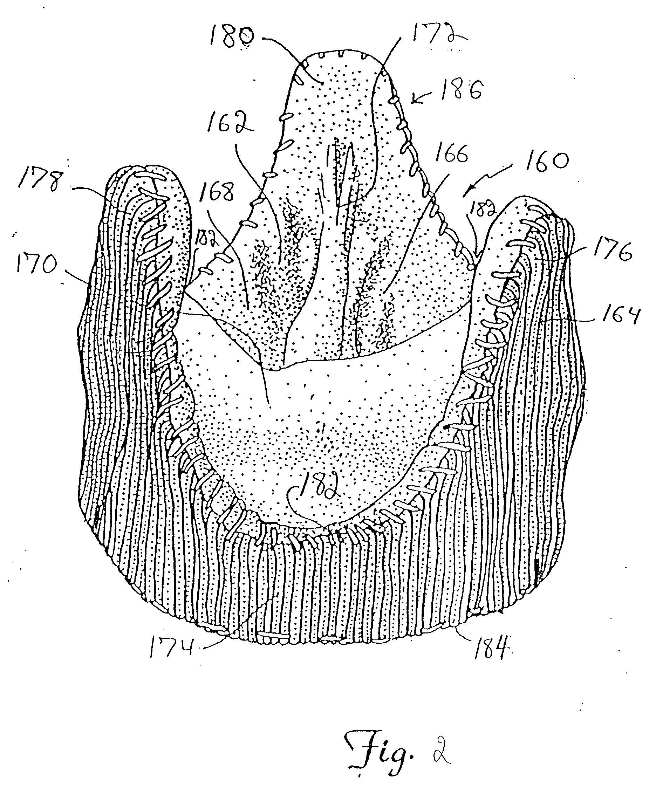

An embodiment of a stentless, tissue heart valve prosthesis is shown in Fig. 2. Heart valve prosthesis 160 includes a harvested tissue valve 162, such as a porcine valve. Prosthesis 160 can further include a fabric cover 164. Valve 162 has three leaflets 166, 168, 170 that meet at coaptation surfaces 172. A generally annular base 174 and three commissure supports 176, 178, 180 support the leaflets. When viewed from the side, three scallops 182 extend between commissure supports 176, 178, 180 along the upper edge 186 of the prosthesis. Lower edge 184 of prosthesis 160 is the inflow end, and upper edge 186 is the outflow end. In this embodiment, lower edge 184 is generally planar, in contrast with the scalloped upper edge 186 of the prosthesis.

Harvested tissue valve 162 and/or fabric cover 164 and/or portions thereof can include an associated stimulation compound. The prosthesis shown in Fig. 2 is suitable for implantation in the aortic or pulmonary positions in a heart. Heart valve prostheses for the mitral and tricuspid position are also suitable for incorporating the materials of the invention. Stented tissue valve prostheses and stented or stentless polymer valve prostheses with flexible leaflets can similarly be formed with an associated stimulation compound.

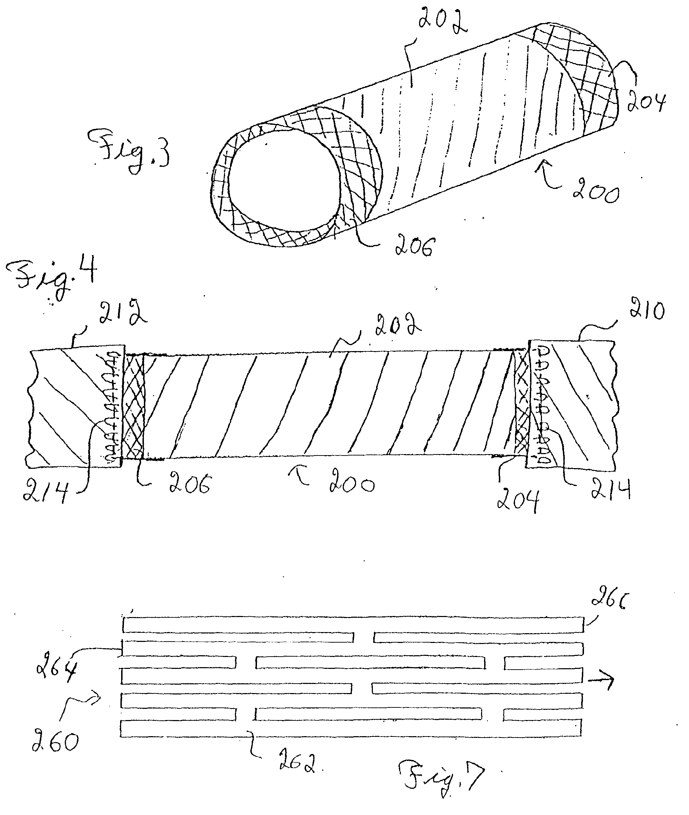

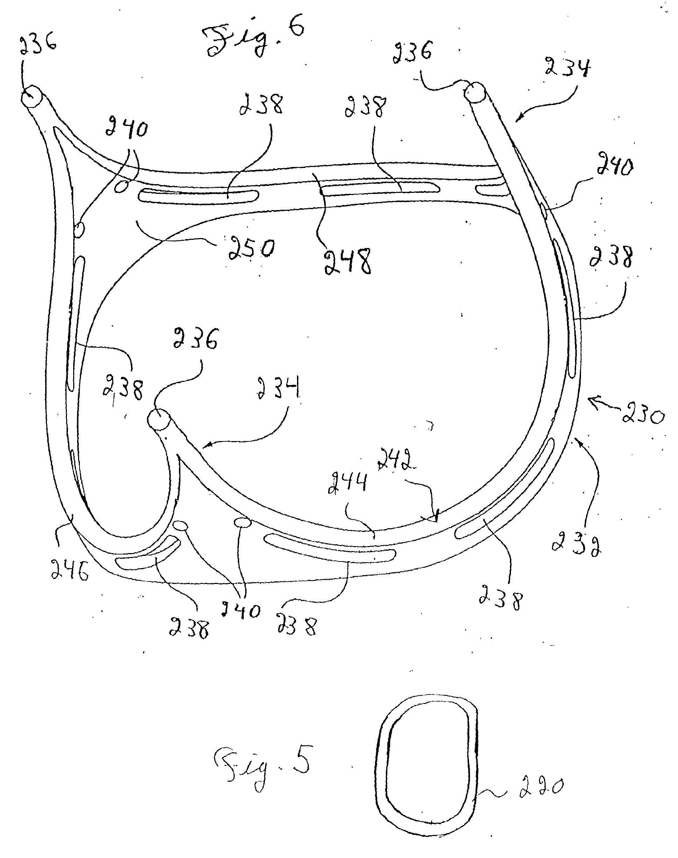

A representative vascular graft 200 is depicted in Fig. 3. Vascular graft 200 includes a flexible tubular structure 202 and optional sewing cuffs 204, 206. Flexible tubular structure 202 can include one or more biocompatible materials, such as tissue, synthetic polymer or combinations thereof. Sewing cuffs 204, 206 can be formed from fabric, such as polyester fabric, tissue or the like. Sewing cuffs 204, 206 assist with the implantation of the prosthesis and may provide reinforcement of the prosthesis at the site of anastomoses, i.e., attachment of the vessel to the graft. A side view of vascular graft 200 attached to natural vessel sections 210, 212 is depieted in Fig. 4. As shown in Fig. 4, suture 214 is used to secure vascular graft 200 to vessel sections 210, 212, although other fastening techniques can be used. One or more of the biocompatible materials and/or portions thereof can have associated stimulation compounds.

Fig. 5 is a top view of an annuloplasty ring. Annuloplasty rings can be used to repair native heart valves to restore valve function and, in particular, to improve leaflet coaptation. In addition, annuloplasty rings can be placed around the annulus of a heart valve prosthesis at implantation to provide further support for the prosthesis. For use in mitral valve repair, an annuloplasty ring 220 can be shaped to have a D-shape, as shown in Fig. 5, although other shapes can also be used. All or a portion of the annuloplasty ring can be constructed from biocompatible materials with associated stimulation compounds.

Fig. 6 is a perspective view of a heart valve stent for supporting flexible leaflets of a heart valve prosthesis. Heart valve stent 230 includes an inflow ring 232, which may be scalloped, and commissure posts 234, which extend to individual post tips 236. Inflow ring 232 has a generally annular configuration. Stent 230 includes openings 238 and retaining holes 240, either or both of which can be used to couple a further biocompatible material to the stent. Stent 230 includes a ridge 242 that extends along the top of stent 230 to form scallops 244, 246, 248 that meet at post tips 236. Flexible support 250 extends from ridge 242 to form inflow ring 232. In some embodiments, ridge 242 is thicker than flexible support 250. Stent 230 can provide a suitable surface for supporting cusps or leaflets of biocompatible material and other components of a heart valve prosthesis.

Preferably, stent 230 has a flexibility approximating the native supporting structure of the patient's tissue. The flexibility of stent 230 can be different at different points along stent 230. Generally, ridge 242 is more rigid than flexible support 250. All or a portion of stent 230 can be formed from biocompatible materials with an associated stimulation compound. Various other designs of heart valve stents can be used with at least a portion thereof formed from a biocompatible material with an associated stimulation compound.

A representative vascular stent design is shown in Fig. 7. Vascular stents can be used to reinforce a blood vessel and/or help to maintain an open channel through the blood vessel. Vascular stent 260 is formed from a biocompatible material 262 with a form consistent with its expandable nature. Generally, stent 260 is transported through the blood vessel to the desired location of deployment and expanded to fit against the walls of the blood vessel. All or a portion of vascular stent 260 can be formed from a biocompatible material with an associated stimulation compound. In some embodiments, the associated stimulation compound is located at or near downstream end 264. Thus, following implantation, the stimulation compound is release into the blood flow in the direction from downstream end 264 to upstream end 266 to contact the entire stent 260 with the stimulation compound. Vascular stents can be formed from metals, polymers and combinations thereof.

While the stimulation compound generally would be associated with a biocompatible material that forms a structural component of the medical device, the stimulation compound can be associated with a portion of biocompatible material that is present solely for the delivery of the stimulation compound. To the extent that the stimulation compound is released from the medical device in a particular embodiment, the medical device functions as a stimulation compound delivery system. However, any biocompatible material that is serving solely as a delivery system for stimulation compounds should not interfere with the functioning of the other components of the medical device. Similarly, the entire medical device can be solely a release platform for the stimulation compound as part of a therapy program without having the medical device serve any other purpose.

### Biocompatible Materials

The medical devices of interest generally include one or more biocompatible materials. Generally, any one or more of the biocompatible materials in the medical device would be suitable for association with a stimulation compound. Relevant biocompatible materials include tissue materials, synthetic materials and combinations thereof. Suitable synthetic material include, for example, metals, ceramics, carbonaceous solids and polymers. Suitable approaches for association of stimulation compounds with the biocompatible material may vary with the type of biocompatible material.

While tissue materials can be particularly suitable for cell colonization, other materials can be suitable for cell colonization and/or association with stimulation compounds. The stimulation compound can be associated with a biocompatible material so that the material undergoes cell colonization. Alternatively, or additionally, the stimulation compound can be associated with a biocompatible material near another biocompatible material suitable for cell colonization. Furthermore, the stimulation compound can be associated with a biocompatible material to stimulate VEGF production and endothelial cell proliferation in the vicinity of the medical device without necessarily promoting cell colonization of the medical device or components thereof.

Synthetic materials can be formed with pores to facilitate delivery of a stimulation compound and/or to facilitate cell adhesion by providing locations for binding of cells to the surface. In some embodiments, the stimulation compound is incorporated into a resorbable material, which is then placed within the pores. As the resorbable material is gradually resorbed, the stimulation compound is released into the surrounding locations. In alternative embodiments, the stimulation compound is combined with a hydrogel polymer and/or an extracellular matrix protein, such as collagen and elastin, which is then placed into the pores. The stimulation compound gradually diffuses out from the hydrogel/extracellular matrix protein material into the surrounding environment. The extent of the porosity can be selected to yield the desired amount of loading of the stimulation compound.

Appropriate ceramic materials include, for example, hydroxyapatite, alumina, and metal/silicon carbides. Relevant carbonaceous solids include, for example, graphite, turbostratic carbon and pyrolytic carbon. Biocompatible metals include, for example, titanium, cobalt, stainless steel, nickel, iron alloys, cobalt alloys, such as Elgiloy®, a cobalt-chromium-nickel alloy, and MP35N, a nickel-cobalt-chromium-molybdenum alloy, and Nitinol®, a nickel-titanium alloy.

Polymeric materials can be fabricated from synthetic polymers as well as purified biological polymers. Appropriate synthetic materials include hydrogels and other synthetic materials that cannot withstand severe dehydration. Suitable polymers also include bioresorbable polymers that are gradually resorbed after implantation within a patient.

Appropriate synthetic polymers include, for example, polyamides (e.g., nylon), polyesters, polystyrenes, polyacrylates, vinyl polymers (e.g., polyethylene, polytetrafluoroethylene, polypropylene and polyvinyl chloride), polycarbonates, polyurethanes, poly dimethylsiloxanes, cellulose acetates, polymethyl methacrylates, ethylene vinyl acetates, polysulfones, nitrocelluloses and mixtures, derivative and copolymers thereof. Bioresorbable synthetic polymers can also be used such as dextran, hydroxyethyl starch, derivatives of gelatin, polyvinylpyrrolidone, polyvinyl alcohol, poly[N-(2-hydroxypropyl) methacrylamide], poly(hydroxy acids), poly(epsilon-caprolactone), polylactic acid, polyglycolic acid, poly(dimethyl glycolic acid), poly(hydroxy butyrate), and similar copolymers. Suitable polymers for the formation of hydrogels include, for example, poly(ethylene glycol), poly(hydroxyethyl methacrylate), partially or fully hydrolyzed poly(vinyl alcohol), poly(vinylpyrrolidone), poly(ethyloxazoline), poly(ethylene oxide)-co-poly(propylene oxide) block copolymers, poloxamines, polyacrylamide, hydroxypropylmethacrylate (HPMA), carboxymethyl cellulose, hydroxyethyl cellulose, methylhydroxypropyl cellulose, polysucrose, hyaluronic acid, dextran, gelatin, sodium alginate, chitosan and mixtures and copolymers thereof. These synthetic polymeric materials can be woven or knitted into a mesh to form a matrix or substrate. Alternatively, the synthetic polymer materials can be molded or cast into appropriate forms.

Biological polymers can be naturally occurring or produced in vitro by fermentation and the like or by recombinant genetic engineering. Recombinant DNA technology can be used to engineer virtually any polypeptide sequence and then amplify and express the protein in either bacterial or mammalian cells. Purified biological polymers can be appropriately formed into a substrate by techniques such as weaving, knitting, casting, molding, extrusion, cellular alignment and magnetic alignment. Suitable biological polymers include, for example, collagen, elastin, silk, keratin, gelatin, polyamino acids, polysaccharides (e.g., cellulose and starch) and copolymers thereof.

Appropriate bioprosthetic tissue materials can be formed from natural tissues, synthetic tissue matrices and combinations thereof. Synthetic tissue matrices can be formed from extracellular matrix proteins that are crosslinked to form a tissue matrix or from synthetic materials, such as polymers, that have or have had viable cells associated with the matrix. Thus, tissue materials have viable cells or structures formed from cells that are no longer present. Suitable polymers and extracellular matrix proteins for incorporation into a synthetic tissue matrix are commercially available. A tissue material can form the entire medical device or it can form one or more portions and/or components of the medical device.

Natural, i.e. biological, tissue material for use in the invention includes relatively intact tissue as well as decellularized tissue. These natural tissues may be obtained from, for example, native heart valves, portions of native heart valves such as roots, walls and leaflets, pericardial tissues such as pericardial patches, amniotic sacs, connective tissues, bypass grafts, tendons, ligaments, skin patches, blood vessels, cartilage, dura mater, skin, bone, fascia, submucosa, umbilical tissues, and the like. Natural tissues are derived from a particular animal species, typically mammalian, such as human, bovine, porcine, seal or kangaroo. These tissues may include a whole organ, a portion of an organ or structural tissue components.

Suitable natural tissues include xenografts, homografts and autografts. These natural tissues generally include collagen-containing material. Natural tissue is typically, but not necessarily, soft tissue. Tissue materials are particularly useful for the formation of tissue heart valve prostheses. The tissue can be decellularized.

Tissue materials can be fixed by crosslinking. Fixation provides mechanical stabilization, for example, by preventing enzymatic degradation of the tissue. Glutaraldehyde, formaldehyde or a combination thereof is typically used for fixation, but other fixatives can be used, such as epoxides, diimides and other difunctional aldehydes. In particular, aldehyde functional groups are highly reactive with amine groups in proteins, such as collagen. Formaldehyde generally does not function alone as a satisfactory crosslinking agent. However, formaldehyde is a common sterilant used to store tissue following glutaraldehyde crosslinking. Contact with aldehydes can introduce some cytotoxicity to tissue, which can be reduced by treatment with alcohol. An improved protocol to remove the cytotoxicity of aldehyde crosslinked tissue is described in copending and commonly assigned U.S. Patent Application serial number 09/480,437 to Ashworth et al., entitled "Biocompatible Prosthetic Tissue," incorporated herein by reference.

### Stimulation Compounds and VEGF

Stimulation compounds stimulate the production of VEGF, which in turn stimulates proliferation of vascular endothelial cells. Thus, release of a stimulation compound in the vicinity of the medical device promotes proliferation of endothelial cells in the vicinity of medical device. HIF is a protein that is known to stimulate VEGF production by promoting transcription of the gene coding for VEGF. Thus, HIF is a suitable stimulation compound. HIF-1 is induced in natural systems by hypoxia. Other proteins that stimulate transcription of the VEGF gene would also be suitable stimulation compounds.

VEGF refers to a family of polypeptides that have been found to preferentially stimulate growth of vascular endothelial cells over other cells, such as smooth muscle cells. Several forms of VEGF have been identified. VEGF polypeptides generally have sequence homology with platelet-derived growth factor, which can alter the migration and proliferation of a variety of cell types. VEGF occasionally has been referred to as vascular permeability factor.

The originally identified form of VEGF has a molecular weight of about 45 to 46 kilodaltons (kDa). This form is a homodimer with each subunit having a molecular weight of about 23 kDa. The c-DNA sequences encoding the human polypeptide (165-amino acids, hVEGF₁₆₅) and the corresponding bovine polypeptide (164-amino acids, bVEGF₁₆₄) have been determined. In addition, variants of the polypeptides with 121-amino acids for the human version (hVEGF₁₂₁) and 120-amino acids for the bovine version (bVEGF₁₂₀) also have been identified. For the corresponding amino acid sequences, see U.S. Patent 5,194,596, to Tischer et al., incorporated herein by reference. Other insoluble variants have been identified with 189 and 206-amino acids, respectively. See, for example, E. Tischer et al., "The human gene for vascular endothelial growth factor. Multiple protein forms are encoded through alternative exon splicing," J. Biol. Chem. 266:11947-11954 (1991) and K. A. Houck et al., "The vascular endothelial growth factor family: identification of a fourth molecular species and characterization of alternative splicing of RNA," Molec. Endocrinology 5:1806-1814 (1991), both incorporated herein by reference.

Another form of VEGF, called VEGF II, is a heterodimer. As isolated from rat glioma cells, the first subunit has 190-amino acids while the second subunit has a 135-amino acid form and an 115-amino acid form. VEGF II is described in EP 0 476 983A, incorporated herein by reference.

A single polypeptide human VEGF, believed to be unnamed, also has been identified. This polypeptide has a molecular weight of roughly 80 kDa. The corresponding cDNA was isolated and a 728-amino sequence was determined from the cDNA sequence. Details of the protein are provided in EP 0 550 296A, incorporated herein by reference.

Still another human growth factor, VEGF2, has been identified from early stage human embryo osteoclastomas, adult heart and several breast cancer lines. VEGF2 has 350 amino acids, of which about 24 amino acids represent a leader sequence. The sequence for VEGF2 is disclosed in WO 95/24473, incorporated herein by reference.

Recently, VEGF-B, another variant of VEGF, has been identified. VEGF-B appears to be associated with heart and skeletal muscles. Full sequences for mouse and human VEGF-B are set forth in U.S. Patent 5,607,918, to Eriksson et al., incorporated herein by reference.

In addition to VEGF variants that are expressed in mammalian cells, viral proteins such as the Tat protein from human immuno-deficiency virus-1 (HIV-1) share sequence homology with VEGF and bind to native VEGF receptors. These properties are described in Albini et al., "The angiogenesis induced by HIV-1 Tat protein is mediated by the Flk-1/KDR receptor on vascular endothelial cells," Nature Medicine 2(12):1371-1375 (1996) and Mitola et al., "Tat-human immunodeficiency virus-1 induces human monocyte chemotaxis by activation of vascular endothelial growth factor receptor-1," Blood 90(4): 1365-1372 (1997), both of which are incorporated herein by reference. Through an interaction with VEGF receptors, a Tat protein stimulates endothelial cell chemotaxis and proliferation. Thus, for the purposes of this application, the Tat protein and other similar viral proteins that bind VEGF receptors are considered a VEGF growth factor.

As described above, a variety of VEGF polypeptides have been identified. Many of these are associated with particular tissues. At least some of the polypeptides have variations based on alternative message splicing, such as hVEGF₁₆₅ and hVEGF₁₂₁. As used in the other sections of this application, "VEGF" refers, without limitation, to all previously identified VEGF polypeptides, such as those described in this section, as well as any future identified VEGF polypeptides that selectively promote the chemotaxis or proliferation of endothelial cells. "VEGF" also refers to polypeptide fragments that maintain their ability to selectively promote the chemotaxis or proliferation of endothelial cells. As noted above, for example, human VEGF₁₂₁ is a naturally occurring fragment of human VEGF₁₆₅. Recombinant human VEGF₁₆₅, human VEGF ₁₂₁, and mouse VEGF are available from R&D Systems of Minneapolis, MN. Similarly, "VEGF" referred to herein includes VEGF proteins modified by chemical additions to the protein molecule by covalent or noncovalent binding.

Using standard molecular biology techniques (see, for example, Sambrook, Fritsch and Maniatis, "Molecular Cloning: A Laboratory Manual," 2nd edition, Cold Spring Harbor Press, (1989)), it is possible to make recombinant modified forms of natural VEGF polypeptides. These straightforward modifications include addition of amino acids on the N-terminus, the C-terminus or both. Also, modifications can be made by substituting amino acids along the polypeptide chain. Some modifications may destroy activity of the protein. It is straightforward to eliminate inactivating modifications by testing for activity in cell culture systems. Active forms of these modified polypeptides are within the general definition of "VEGF."

Transcription of the VEGF genes is carefully controlled in mammals. Evidence suggests that the VEGF genes are regulated by both a promoter and an enhancer element. In some embodiments, appropriate stimulation compounds can interact with the enhancer element and promote transcription of the VEGF gene. The enhancer element for VEGF has been termed a hypoxia response element. The hypoxia response element (HRE) is a DNA segment adjacent the gene. HIF and related compounds are observed to be effective at inducing transcription of the VEGF genes.

Transcription of several genes, including VEGF, is known to be controlled by a promoter and a hypoxia response element. Several HREs are known including, for example, erythropoietin HRE, muscle pyruvate kinase HRE, β-enolase HRE, endothelin-1 HRE and metallothionein II HRE. Without activation of the HRE, little, if any, transcription takes place. The HRE's are described further in U.S. Patent 6,218,179 to Webster et al., entitled "Tissue Specific Hypoxia Regulated Constructs," incorporated herein by reference.

HIF-1 is a polypeptide that has been observed to induce transcription of hypoxia inducible proteins, such as VEGF. While not wanting to be limited by theory, HIF-1 may be functioning by activating an HRE. HIF-1 is a heterodimer of subunits HIF-1α and HIF-1β. HIF-1α is unique to HIF-1. The concentration of RNA associated with each subunit increases in response to hypoxia conditions. Different forms or the same form of HIF-1 may be associated with different VEGF genes found in humans. Any different forms of HIF may likely, but not necessarily, have sufficient sequence homology with identified forms of HIF in order to reasonably predict function. Any of these forms of HIF would be suitable as stimulation compounds.

VEGF was discovered in association with tumor tissue. Tumor tissue was observed to secrete VEGF to promote vascular permeability. VEGF production is stimulated in a cascade response to hypoxia, acidosis and hypoglycemia. Hypoxia stimulates production of HIF-1α, which in turn stimulates production of VEGF. Responses based on acidosis, hypoglycemia and any other condition that promotes the production of VEGF presumably activate the enhancer/hypoxia response element, HIF may or may not be involved in these other responses. Other proteins that activate the enhancer/hypoxia response element for VEGF that may be later identified would also be suitable as stimulation compounds.

Several features of the domains of HIF-1α have been elucidated. For example, the minimum domains for transactivation are at amino acid residues 531-575 and 786-826. A mutant form of HIF-1 has been discovered that is more stable under non-hypoxia conditions. This form has an HIF-1α subunit with amino acids 392-428 deleted and amino acid 551 changed from a serine to any other amino acid and amino acid 552 changed from a threonine to any other amino acid. These mutants would be particularly suitable for use in the present invention. The mutant forms of the protein can easily be obtained from the gene sequence using standard molecular biology techniques. These mutants are described in detail in U.S. Patent 6,124,131 to Semenza (the Semenza '131 patent), entitled "Mutant Hypoxia Inducible Factor-1 HIF-1," incorporated herein by reference.

Furthermore, it is well known that many point mutations, amino acid additions and/or amino acid deletions do not alter protein function or decrease protein function sufficiently to eliminate desired VEGF stimulation. Mutant HIF proteins with suitable protein function for stimulation of the enhancer/hypoxia response element associated with a VEGF gene can be used in the present invention.

Nucleic acid compounds coding for proteins that function as stimulation compounds can be formulated into stimulation compounds themselves using gene therapy techniques. For example, the gene coding for the desired protein in a C-DNA form or a RNA form can be introduced as a stimulation compound. The nucleic acids can be introduced into a colloidal dispersion system, such as oil-in-water emulsions, micelles, and liposomes. Liposomes form artificial membrane vesicles that can include, for example, phospholipids, sterols, such as cholesterol, and lipids, such as phosphatidyl-glycerol, phosphatidylcholine, phosphatidylserine, phosphatidylethanolamine, sphingolipids, cerebrosides and gangliosides. The liposomes can be used as the delivery vehicle for the stimulation compound.

Alternatively, the gene can be introduced into a suitable viral vector, such as adenoviruses, adeno-associated virus, herpes virus, or retroviruses, including, for example, Moloney murine leukemia retrovirus, Harvey murine sarcoma virus, murine mammary tumor virus, Rous sarcoma virus, gibbon ape leukemia virus and other murine retroviruses and avian retroviruses. Recombinant retroviruses can be formed into infectious delivery vectors by combining the retroviruses with plasmids encoding the viral structural genes in helper cell lines. The resulting vector viron can be delivered as a stimulation compound.

In summary, stimulation compounds generally result in the production of VEGF. Suitable stimulation compounds include proteins that activate the enhancer/hypoxia response element associated with the genes for VEGF. Specific stimulation compounds include, for example, HIF, mutants, fragments, and other modified forms of HIF that are effective to stimulate the hypoxia response element of VEGF genes. In addition, nucleic acids in a suitable delivery vehicle, such as a viral vector or a colloidal dispersion system, can be used as a stimulation compound.

### Association of Stimulation Compound

The stimulation compounds can be associated with a biocompatible material of a medical device. The stimulation compound generally is releasably associated with the biocompatible material such that the stimulation compound is gradually released into the fluids and/or tissue surrounding the medical device. The stimulation compound can be effective to induce VEGF production by the patient's cells in the vicinity of the medical device. Correspondingly, the production of VEGF in the vicinity of the medical device promotes the proliferation of endothelial cells. The stimulation compound can be associated with the biocompatible material by direct association, chemical bonding, adhesive binding and/or incorporation into the matrix of the biocompatible material. These approaches can be combined with microencapsulation.

Suitable binding approaches may depend on whether the stimulation is bound reversibly and, if it is bound reversibly, the desired rate of release of the stimulation compound. Generally, the amount of stimulation compound associated with a prosthesis depends on the release rate, the lifetime of the activity of the stimulation compound, the desired length of time of delivery as well as potential practical limits on loading. In some embodiments, the prosthesis includes at least about 1 milligram (mg) of stimulation compound, alternatively at least about 10 mg of stimulation compound, in other embodiments from about 20 mg to about 500 mg, and in other embodiments, from about 25 mg to about 100 mg. A person of ordinary skill in the art will recognize that other ranges within these explicit ranges are contemplated and are within the present disclosure.

In particular, for some embodiments, the stimulation compound may have to penetrate into a cell of the patient to be effective, such as by binding to a cell's DNA to stimulate expression of the VEGF gene or by expressing a protein that is itself a stimulation compound. For these embodiments, the binding of the stimulation compound to the medical article is relatively weak such that released stimulation compound can enter the appropriate cells. Furthermore, appropriately weak binding of the stimulation compound to the medical device can result in the dissociation of the compound from the medical article over a relatively short time frame. The relatively rapid release of the stimulation compound can be particularly desirable if the stimulation compound results in colonization of the medical device with viable cells or if the presence of endothelial cells is particularly beneficial during the healing process after the implantation of the medical device. In contrast, a slower release rate results in a therapeutic effect over a longer period of time. The magnitude of the association of the stimulation compound with the medical device can be adjusted to obtain a desired release rate.

As an alternative to relatively weak binding of the stimulation compound to the medical device while providing for release of the stimulation compound, the bond between the stimulation compound and the medical device can include a linkage that is sensitive to metabolic or enzymatic activity within the patient. For example, the linkage between the stimulation compound and the medical device can include peptide sequences that are sensitive to proteolysis by enzymes to which the medical device is expected to be exposed in vivo. Thus, the stimulation compound will gradually be released from the medical device over a time frame determined by the efficiency of the metabolic or enzymatic activity.

In other alternative embodiments, microencapsulation can be used for further control over the release rate. The microencapsulated material may or may not be strongly bonded to the structure of the medical device. Nevertheless, the encapsulated material can gradually release the stimulation compound over a period of time.

Direct association entails combining the biocompatible material with a solution of the stimulation compound, without the use of an additional chemical binder. Due to direct contact, the stimulation compound can bind with the biocompatible material, possibly due to chemical bonding with free functional groups in the biocompatible material. For direct association of the stimulation compound to the biocompatible material, the biocompatible material or a portion thereof is combined with a solution of one or more stimulation compounds at a concentration generally from about 0.1 nanomolar (nM) to about 1 micromolar (µM) and preferably from about 0.5 nM to about 10 nM.

In embodiments involving tissue materials, the solution containing the stimulation compound preferably is buffered at a near physiological pH ranging from about 6.0 to about 8.5, and more preferably ranging from about 6.9 to about 7.5. Suitable buffers can be based on, for example, the following compounds: phosphate, borate, bicarbonate, carbonate, cacodylate, citrate, and other organic buffers such as tris(hydroxymethyl) aminomethane (TRIS), N-(2-hydroxyethyl) piperazine-N'-(2-ethanesulfonic acid) (HEPES), and morpholine propanesulphonic acid (MOPS).

Alternatively, the stimulation compound can be associated with the biocompatible material through the use of a binder or adhesive. The stimulation compound associates with the biocompatible material due to incorporation into the structure of the adhesive when the adhesive cures. The stimulation compound and the adhesive form a coating on the biocompatible material, generally with some penetration into the biocompatible material. If the adhesive is bioresorbable, the stimulation compound is released as the adhesive is resorbed. Suitable adhesives include, for example, biologic glues such as fibrin glue, and the like. Fibrin glue can be formed from the polymerization of fibrinogen and thrombin. Suitable fibrin glues are available from, for example, Immuno AG, Austria.

To apply the stimulation compound with fibrin glue to a biocompatible material, a small amount of thrombin can be absorbed into the biocompatible material. The stimulation compound can be mixed with a solution containing fibrinogen to yield a fibrinogen-stimulation compound solution with a concentration of the stimulation compound preferably ranging from about 0.1 nM - 1 µM. Then, the solution can be brushed or sprayed over the surface of the biocompatible material with absorbed thrombin, or the biocompatible material with absorbed thrombin can be dipped into the fibrinogen-stimulation compound solution. The coating can be applied to all or just a portion of the biocompatible material.

With synthetic biocompatible materials, the stimulation compound also can be incorporated into the material when the biocompatible material is formed. When incorporated into the matrix of the biocompatible material, the stimulation compound can be located in interstitial spaces within the biocompatible material. In particular, at an appropriate time in the preparation of the biocompatible material, an amount of the stimulation compound can be mixed with the components of the biocompatible material to form a blend. The blend is formed into a biocompatible material with the stimulation compound within its structure. The biocompatible substrate can include several regions and/or layers such that the stimulation compound is only distributed within a subset of the regions and/or layers of the biocompatible material. The stimulation compound can be released from the material either by diffusion, if liquids can penetrate into the biocompatible material, or by resorption of the biocompatible material, for example, as a result of forming the biocompatible material partially or completely from a bioresorbable polymer.

In other embodiments, the association of a stimulation compound with a biocompatible material involves chemical binding initiated by a selected chemical reagent and/or a chemical binding agent. In contrast with the use of an adhesive, chemical binding involves specific molecular interactions with biocompatible material compositions, rather than a collective adhesion. Chemical binding can involve covalent bonding, noncovalent chemical interactions, or a combination of both covalent and noncovalent interactions. Noncovalent chemical interactions include, for example, hydrogen bonds, van der Waals interactions, ionic interactions and/or molecular rearrangements, which characterize specific binding interactions, such as antibody-antigen interactions, protein-receptor binding and enzyme-substrate associations. In other words, reactants or binding agents are used to form a chemical association between the stimulation compound and the biocompatible material, possibly involving a linker molecule.

As noted above, in some embodiments it may be desirable for the bonding of the stimulation compound to be relatively weak such that the bonding is reversible on a pre-selected desired time frame. The length of the desired time frame depends on the binding strength of the stimulation compound to the biocompatible material, the amount of stimulation compound present, the rate of inactivation by degradative enzymes, and possibly other factors. The chemical bonding of the stimulation compound to the medical device can be adjusted by selection of the binding approach and/or binding compositions to yield the desired equilibrium constants with respect to unbound stimulation compound.

The chemical binding of the stimulation compound with the biocompatible material can involve covalent bonding to the biocompatible material with reactive agents such as glutaraldehyde or other suitable crosslinking agents. This is an especially suitable procedure for the strong binding of polypeptide ligands and antibodies with biocompatible material formed from tissue or other polymers with suitable functional groups. A typical procedure for the crosslinking of the stimulation compound to the surface of a tissue makes use of glutaraldehyde, which crosslinks proteins by way of two aldehyde groups.

Since glutaraldehyde is typically used for fixation of tissue, the crosslinking to bind the stimulation compound to a tissue can be performed simultaneously with fixation of the tissue. Alternatively, crosslinking to covalently bond the stimulation compound with a tissue or synthetic substrate can be performed as a separate step before or after the completion of a fixation process, assuming a fixation step is performed. Other chemical reagents for covalent bonding of a stimulation compound to a biocompatible material include, for example, epoxies and other difunctional aldehydes, such as glyoxal.

Other polyfunctional linkers can be designed to react with specific functional groups both in the stimulation compound and in the biocompatible material, such that the linkers generally bond simultaneously with the stimulation compound and biocompatible material. The linkers incorporate one or more functional groups that covalently bond to corresponding functional groups on the biocompatible material and one or more functional groups that covalently bond to functional groups on the stimulation compound. Thus, the linker covalently links the stimulation compound to the biocompatible material.

Alternatively, chemical binding of the stimulation compound to the biocompatible material can involve specific binding interactions. The specific binding interactions can be used to target specific locations within the biocompatible material. The targeting of specific locations in the biocompatible material can be useful, for example, if specific locations are particularly susceptible to or desirable for colonization by endothelial cells or disruption of the flow. An example of a possible target location includes sewing cuffs of a prosthesis, such as a mechanical heart valve.

A method of targeting a particular location involves the use of linkers that target specific cellular or extracellular binding sites within a natural tissue or other biocompatible material. In certain embodiments, the linker is covalently bonded to the stimulation compound, and the linker associates with the biocompatible material by a plurality of non-covalent interactions, such as antibody-antigen interactions. Alternatively, the linker can be covalently bonded to the biocompatible material and the stimulation compound can be associated with the linker by a plurality of non-covalent interactions. A variety of commercially available antibodies and other specific binding reagents may be used as linkers. Alternatively, antibodies and other specific binding reagents can be prepared by conventional techniques.

Covalent bonds can be subject to equilibrium conditions similar to other types of associations. The strengths of the covalent bonds can be selected to provide a desired equilibrium between the bound and unbound states. In the unbound state in vivo, the stimulation compound is released into the body fluid such that rebinding is unlikely. Thus, the use of appropriately weak covalent bonding can be a suitable approach for associating the stimulation compound with a biocompatible material with the gradual release of the stimulation compound following implantation of the corresponding medical device.

A stimulation compound having an attached antibody/ligand or any other comparable targeting molecule/linker for binding to a biocompatible material is considered a stimulation compound for the purposes of the present application. Similarly, an engineered chimera of the stimulation compound and the targeting molecule is considered a stimulation compound for the purposes of the present application. The chemical binding of compounds to antibodies/ligands as well as the development of chimeras is well established, especially where the compound is a protein.

In an alternative embodiment, photochemical coupling can be used for covalent coupling. Photochemical coupling is based on the use of high energy light, e.g., ultraviolet light, to form reactive intermediates of certain functional groups. These reactive intermediates can form carbon-carbon bonds between two compositions. Aryl ketone functional groups are particularly useful in this respect.

Photochemical coupling can be used for attachment of stimulation compounds to tissue or other biocompatible materials. For a general discussion of photochemical coupling, see, for example, Dunkirk et al., J. Biomaterials Applications 6:131-156 (1991), incorporated herein by reference. The tissue may or may not be separately crosslinked since the photochemical coupling generally also crosslinks the tissue, i.e., photofixation. Photochemical coupling can be used to attach a linker to the biocompatible material either before, after, or during binding of the linker to a stimulation compound.

In other embodiments, microencapsulation is used to control the release of the material. In microencapsulation, the stimulation compound is combined with an encapsulating material that gradually releases the stimulation compound into the neighboring environment. The encapsulated material, for example in the form of microspheres can then be bound to the biocompatible material of the medical device using the approaches described above, i.e., direct association, chemical bonding, adhesive bonding and/or incorporation into the matrix of the material. The encapsulation material can be, for example, a resorbable polymer, which is combined with the stimulation compound and formed into microspheres.

An improved approach for the microencapsulation and controlled release of nerve growth factors is described in U.S. Patent 6,113,947 to Cleland et al., entitled Controlled Release Microencapsulated NGF Formulation," incorporated herein by reference. This approach can be adapted for the controlled release of stimulation compounds. These formulations are based on "biodegradable" polymers incorporating hydrocarboxylic acids in polymers or copolymers. A metal salt is added to solutions containing the bioresorbable polymer. The growth factor is also added to the solution with the bioresorbable polymer. The microencapsulated particles are formed from the solutions of polymer, metal salt and protein by spray drying, phase separation or other drying methods.

Empirical adjustments can be made to ensure that the activity of the stimulation compound is not significantly impaired when the stimulation compound is associated with the biocompatible material. In particular, the conditions used to associate the stimulation compound with the biocompatible material can be altered to ensure appropriate activity of the stimulation compound, for example, by changing the association approach or the particular binders used. The activity of the stimulation compound can be verified in vitro, for example, in a tissue culture system with appropriate cell types to ensure that the stimulation compound is effective at stimulating the production of VEGF.

The activity of the stimulation compound generally has a finite lifetime, preferably long enough to accomplish the intended purpose of the stimulation compound. Loss of activity may be due to proteolytic activity, other biological degradation processes and/or denaturing of the materials due to other chemical processes. Thus, storage times, release rates and other time scales can be selected to account for the activity lifetime of the stimulation compound as determined by efficacy testing.

### Other Biologically Active Compounds

Along with the stimulation compound, the medical device can be associated with a biological response modifier that is either released into the flowing body fluids or bound to the medical device to affect the interaction of the fluid and body tissue with the medical device. In particular, the biocompatible material can be treated to stimulate the association of desirable cells with the biocompatible material, to promote the proliferation of cells at or near the medical device and/or to reduce calcification of the medical device following implantation. For example, a biocompatible material can be associated with one or more growth factors, such as vascular endothelial growth factor (VEGF) and fibroblast growth factor, and/or attraction compounds that recruit cells, including precursor cells, to the biocompatible material. Other suitable biological response modifiers include, for example, anticalcification agents.

While the stimulation compound stimulates the generation of VEGF in the vicinity of the biocompatible material, it may be desirable to also have VEGF associated with the biocompatible material. For example, if the stimulation compound is released, the stimulation compound can stimulate the production of VEGF near the medical device that promotes the proliferation of endothelial cells in the vicinity of the medical device. Then, VEGF associated with the biocompatible material associated with the stimulation compound and/or another biocompatible material of the medical device can stimulate the colonization of the medical device, which is more effective due to the local proliferation of endothelial cells as a result of the released stimulation compound. Thus, the combined use of an associated stimulation compound and associated VEGF can have a synergistic effect with respect to promoting the colonization of the biocompatible material. In addition, the stimulation compounds may result in other beneficial biological responses other than VEGF production that can have a beneficial effect with respect to blood flow in the vicinity of the medical device.

The use of VEGF without a stimulation compound in the production of prostheses has been described further in copending and commonly assigned U.S. Patent Applications 09/014,087 to Carlyle et al., entitled "Prostheses With Associated Growth Factors," and 09/186,810 to Carlyle et al., entitled "Prostheses With Associated Growth Factors," both of which are incorporated herein by reference.

In addition, the use of fibroblast growth factors in addition to the stimulation compounds may encourage the colonization of the medical device by fibroblasts, endothelial cells and similar cells. While the fibroblast growth factor stimulates fibroblast and possibly other cell proliferation at the medical device, the stimulation compound can stimulate the proliferation of endothelial cells in the vicinity of the medical device. As with the association of VEGF, the association of a fibroblast growth factor along with the stimulation compound can produce a synergistic effect with respect to providing beneficial effects on cell colonization and/or flow in the vicinity of the biocompatible material. Furthermore, it may be desirable to associate the stimulation compound with different portions of the medical device than the portions associated with growth factors, with or without some overlap.

For the attraction of precursor cells, desirable precursor cells include both stem cells and progenitor cells that have the potential to differentiate into the cells of interest, including fibroblasts or endothelial cells. Some precursor cells circulate in a patient's blood stream, while others may be mobilized from other sites in the body or infused into the circulation. These precursor cells are thus available to colonize suitable blood contacting substrates. Suitable precursor cells can be selected from the blood stream and associated with a substrate that serves as the foundation for a viable prosthetic tissue. To initiate the colonization by the precursor cells, an attraction compound can be associated with a biocompatible material of the medical device. Circulating precursor cells may be removed from circulation by the attraction compound and become associated with the substrate. The use of attraction compounds, such as antibodies and ligands, to associate precursor cells with a substrate is described further in copending and commonly assigned U.S. Patent Application Serial No. 09/203,052 to Carlyle, entitled "Substrates For Forming Synthetic Tissue," incorporated herein by reference.

The association of biological response modifiers, e.g., a growth factor and/or an attraction compound, with a biocompatible material each may involve direct attachment, application of a coating including an adhesive or binder, or chemical binding involving a binding agent in addition to the attraction compound/response modifier. These approaches are described further above.

Also, it may be desirable to contact the tissue with one or more calcification reducing agents. Suitable calcification reducing agents include detergents (e.g., sodium dodecyl sulfate), toluidine blue, diphosphonates, and multivalent cations, especially Al⁺³, Mg⁺² or Fe⁺³, or corresponding metals that can oxidize to form the multivalent metal cations. The effectiveness of AlCl₃ and FeCl₃ in reducing calcification of crosslinked tissue is described in U.S. Patent 5,368,608 to Levy et al., entitled "Calcification-Resistant Materials and Methods of Making Same Through Use of Multivalent Cations," incorporated herein by reference. The association of anticalcific elemental metals is described in copending and commonly assigned U.S. Patent Application serial number 09/017,185 to Ogle et al., entitled "Calcification-Resistant Medical Articles," incorporated herein by reference.

### Production Of Medical Devices

In general, the stimulation compound can be associated with the biocompatible material before, during or after assembly of the medical device, if any assembly is required. A suitable ordering of the steps may depend on the nature of the biocompatible material and the association approach used for the stimulation compound. Similarly, whether or not the stimulation compound is associated with the entire medical device or only a fraction thereof may influence the order of processing steps.

If the stimulation compound is incorporated into the biocompatible material, the incorporation is performed generally during the formation of the biocompatible material. Any further assembly of the biocompatible material into the medical device is performed with the stimulation compound present. In some embodiments, the stimulation compound may only be associated with a portion of the medical device. In these embodiments, it may be desirable to first associate the stimulation compound with the biocompatible material corresponding to the portion of the medical device to include the stimulation compound and then complete fabrication of the medical device, although the association of the stimulation compound can be directed to a portion of the medical device, for example, using an adhesive.

With respect to one embodiment of particular interest, the medical device is a mechanical heart valve prosthesis. The stimulation compound is associated with the sewing cuff, such as sewing cuff 118 in Fig. 1. The sewing cuff generally is formed from a fabric, such as a polyester fabric or a bioresorbable polymer. The stimulation compound can be associated with the sewing cuff by a variety of approaches including, for example, direct association, adhesive association, incorporation into the polymer material and/or various forms of chemical bonding. Generally, the stimulation compounds arc associated with the sewing cuff material prior to attaching the sewing cuff to the mechanical valve.

Other embodiments of particular interest involve prostheses with tissue material. The stimulation compound is associated with all or a portion of the tissue material. Prostheses of particular interest include tissue heart valve prostheses, such as the prosthesis shown in Fig. 2. The stimulation compound can be associated with the tissue by a variety of approaches, such as direct association, adhesive association and chemical bonding. Generally, following association of the stimulation compound with the tissue, any other components, such as fabric covers, reinforcements, stents and the like, are assembled with the tissue into the completed prosthesis.

### In vitro Attachment of Endothelial Cells And Cell Culturing

Growth of viable endothelial cells on prostheses prior to implantation into a patient can be promoted in vitro by joining the stimulation compound with a substrate. In order to reduce the possibility of transplant rejection, the endothelial cells used for in vitro endothelialization preferably are autologous cells, i.e., cells from the ultimate recipient. Suitable endothelial cells could be harvested from, for example, adipose tissue of the patient. The harvesting process can involve liposuction followed by collagenase digestion and purification of microvascular endothelial cells. A suitable process is described further in S. K. Williams, "Endothelial Cell Transplantation," Cell Transplantation 4:401-410 (1995), incorporated herein by reference and in U.S. Patents 4,883755, 5,372,945 and 5,628,781, all three incorporated herein by reference. Purified endothelial cells can be suspended in an appropriate growth media such as M199E (e.g., Sigma Cell Culture, St. Louis, MO) with the addition of autologous serum.

Prosthetic material with associated stimulation compound can be incubated in a stirred cell suspension for a period of hours to days to allow for endothelial cell seeding. Cell seeding provides random attachment of endothelial cells that can proliferate to coat the surface of the prosthetic substrate either before or after implantation into the patient. Alternatively, the prosthetic substrate can be incubated under a pressure gradient for a period of minutes to promote cell sodding. A suitable method for cell sodding can be adapted from a procedure described for vascular grafts in the S. K. Williams article, supra. Cell sodding can produce a monolayer of cells on the surface of the prosthetic tissue.

In addition, the prosthetic material can be placed in a culture system where the patient's endothelial cells are allowed to migrate onto the surface of the prosthetic substrate from adjacent tissue culture surfaces. If either attachment or migration of endothelial cells is performed under conditions involving physiological shear stress, then the endothelial cells colonizing the surface of the substrate may express appropriate adhesion proteins that allow the cells to adhere more tenaciously following implantation.

In other embodiments, a portion of biocompatible material with associated stimulation compounds is placed in a cell culture system as a time release agent to gradually release stimulation compound into the cell culture. Stimulation compound could be desirable in the cell culture system to provide a constant regeneration of VEGF through cellular activity.

### Storage, Distribution and Use

Following association of a stimulation compound with a biocompatible material, the biocompatible material, possibly formed into a prosthesis, can be stored. The biocompatible material preferably would not have ingrowth of viable cells if the tissue is intended for longer term storage. Preferred storage techniques minimize the risk of microbial contamination. For example, the biocompatible material can be stored in a sealed sterile container, with sterile buffer and/or saline solution if appropriate. Some biocompatible tissue, such as tissue and hydrogel, may need to be stored in a moist or hydrated state to prevent unwanted modification of the material. In addition, some stimulation compounds may be stored in a moist state to maintain activity of the stimulation compound.

In a sealed container, the biocompatible material is not subjected to a continuous supply of fluids. Nevertheless, consideration should be given to possible loss during storage of stimulation compounds and/or other protein modifiers from the material, or loss during storage of activity of any stimulation compound or protein modifiers, or the remaining viability of cells associated with the biocompatible material. If excessive loss of desired activity is a possibility, the storage time can be limited appropriately to keep the loss to an acceptable level.

For distribution, the biocompatible material generally is placed in sealed and sterile containers. To prevent undesirable contamination of the biocompatible material, care must be taken to ensure acceptable levels of sterility. To ensure maintenance of acceptable levels of sterility, the biocompatible material/prosthesis can be transferred to the sterile container using accepted aseptic protocols. Furthermore, the container with the biocompatible material/prosthesis can be sterilized using radiation before or after scaling the container. For example, the use of electron beam irradiation to sterilize crosslinked tissue is described in U.S. Patent 5,989,498 to Odland, entitled "Electron-Beam Sterilization of Biological Materials," incorporated herein by reference. The containers can be dated such that the date reflects the maximum advisable storage time.

The containers generally are packaged with instructions for the use of the medical devices along with desired and/or required labels. The containers are distributed to health care professionals for surgical implantation of the prostheses. The implantation is performed by a qualified health care professional. The surgical implantation generally involves the replacement of damaged or diseased tissue with the prosthesis or the implantation of a catheter or the like to provide suitable access into the patient.

In vitro affiliation of cells with a biocompatible material with associated stimulation compound, if performed, can be performed at a hospital where the patient's cells can be removed for use in a cell culture system. The harvested cells can be contacted with the biocompatible material in a cell culture system to associate the cells with the biocompatible material. Thus, a prosthesis with viable cells is formed based on cells from the patient prior to implantation. Longer-term storage of the colonized biocompatible material is avoided by performing the cell culturing at the hospital where the patient is located.

The embodiments described above are intended to be exemplary and not limiting. Additional embodiments are within the claims. Although the present invention has been described with reference to preferred embodiments, workers skilled in the art will recognize that changes may be made in form and detail without departing from the spirit and scope of the invention.

## Claims

1. A medical device comprising a stimulation compound that stimulates production of VEGF, the medical device being an implantable medical device, a catheter, a dressing or a surgical instrument.

2. The medical device of claim 1 wherein the stimulation compound comprises a polypeptide.

3. The medical device of claim 2 wherein the polypeptide comprises hypoxia-inducible factor 1.

4. The medical device of claim 2 wherein the polypeptide comprises hypoxia-inducible factor 1-alpha.

5. The medical device of claim 2 wherein the polypeptide comprises a mutant form of hypoxia-inducible factor 1-alpha that is more stable than the native form under non-hypoxia conditions.

6. The medical device of claim 2 wherein the polypeptide binds to the VEGF hypoxia response element.

7. The medical device of claim 1 wherein the stimulation compound stimulates transcription of VEGF.

8. The medical device of claim 1 wherein the medical device comprises a heart valve prosthesis.

9. The medical device of claim 8 wherein the valve has flexible leaflets.

10. The medical device of claim 9 wherein the flexible leaflets comprise a polymer.

11. The medical device of claim 9 wherein the flexible leaflets comprise tissue.

12. The medical device of claim 11 wherein the stimulation compound is associated with the tissue leaflets.

13. The medical device of claim 9 wherein the heart valve prosthesis further comprises a support structure supporting the leaflets and a sewing cuff.

14. The medical device of claim 13 wherein the sewing cuff comprises fabric and wherein the fabric is associated with the stimulation compound.

15. The medical device of claim 13 wherein the stimulation compound is associated with the support structure supporting the leaflets.

16. The medical device of claim 8 wherein the valve has a rigid pivoting occluder.

17. The medical device of claim 1 comprising a sewing cuff wherein the stimulation compound is associated with the sewing cuff.

18. The medical device of claim 1 wherein the medical device comprises a vascular graft.

19. The medical device of claim 1 wherein the medical device comprises a polymer material in which VEGF production stimulator is incorporated within the polymer material.

20. The medical device of claim 1 wherein the prosthesis comprises tissue.

21. The medical device of claim 20 wherein the tissue is crosslinked.

22. The medical device of claim 20 wherein the tissue is uncrosslinked.

23. The medical device of claim 1 wherein the prosthesis comprises at least about 10 mg of stimulation compound.

24. The medical device of claim 1 wherein the prosthesis comprises at least about 100 mg of stimulation compound.

25. The medical device of claim 1 wherein the medical device is a vascular stent comprising a biocompatible material.

26. The medical device of claim 1 wherein the stimulation compound is releasably bound to a material of the medical device.

27. The medical device of claim 26 wherein the stimulation compound is adhesively bonded.

28. The medical device of claim 26 wherein the stimulation compound is covalently bonded.

29. The medical device of claim 26 wherein the stimulation compound is microencapsulated.

30. The medical device of claim 1 wherein the medical device comprises an annuloplasty ring.

31. A method for producing a medical device, the method comprising associating a stimulation compound with a biocompatible material.

32. The method of claim 31 wherein associating the stimulation compound with the biocompatible material comprises direct association.

33. The method of claim 31 wherein associating the stimulation compound with the biocompatible material comprises chemical bonding.

34. The method of claim 31 wherein associating the stimulation compound with the biocompatible material comprises adhesive bonding.

35. The method of claim 31 wherein associating the stimulation compound with the biocompatible material comprises incorporating the stimulation compound into the matrix of the biocompatible material.
